# EUROPEAN PATENT APPLICATION

(11) **EP 4 613 230 A1**
(43) Date of publication of application: **10.09.2025**
(21) Application number: 24161582.2
(22) Date of filing: 05.03.2024
(51) Int. Cl.: A61B 90/00, A61B 90/94, A61B 34/20, A61B 17/00

(54) **MEDICAL MARKER ELEMENT, MEDICAL INSTRUMENT AND MEDICAL SYSTEM**

(71) Applicant: Aesculap AG, 78532 Tuttlingen (DE); Koninklijke Philips N.V., 5656 AG Eindhoven (NL)
(72) Inventor: Barth, Juergen, 78588 Denkingen (DE); Wagner, Cécile, 78532 Tuttlingen (DE); Pirson, Romain, 5684 PC Best (NL); van Delft, Huub, 5684 PC Best (NL); Koen, Gerrits, 5684 PC Best (NL); Steenbakkers, Mark, 5684 PC Best (NL); Phoon, Kin Fatt, 5684 PC Best (NL)
(74) Representative: Hoeger, Stellrecht & Partner Patentanwälte mbB

(57) **Abstract**

The invention relates to a medical marker element which is formed for the releasable connection to a medical instrument, the medical marker element being optically detectable for determining a position and/or orientation of the medical marker element in space, in particular in an operating room, wherein the medical marker element comprises a marker element interface which is formed for the connection to a corresponding instrument interface of the medical instrument in a detection position, wherein the marker element interface and the instrument interface are in engagement in the detection position in a defined manner and are out of engagement in a cleaning position, wherein the marker element comprises a sleeve surrounding a shaft portion of a shaft of the medical instrument.

Moreover, improved medical instruments and medical systems are proposed.

## Description

The present invention relates to a medical marker element which is formed for the releasable connection to a medical instrument, the medical marker element being optically detectable for determining a position and/or orientation of the medical marker element in space, in particular in an operating room, wherein the medical marker element comprises a marker element interface which is formed for the connection to a corresponding instrument interface of the medical instrument in a detection position, wherein the marker element interface and the instrument interface are in engagement in the detection position in a defined manner and are out of engagement in a cleaning position.

Further, the present invention relates to a medical instrument comprising a shaft, wherein the shaft has a shaft portion, which defines a shaft longitudinal axis of the instrument, and wherein the medical instrument has an instrument interface for releasably connecting to a medical marker element interface of a medical marker element, which is optically detectable for detecting a position and/or orientation of the medical marker element and the medical instrument connected thereto in space, in particular in an operating room, wherein the instrument interface and the marker element interface are in engagement in a detection position in a defined manner and are out of engagement in a cleaning position.

Moreover, the present invention relates to a medical system comprising at least one medical instrument and at least one medical marker element, the at least one marker element being releasably connectable to the at least one medical instrument in a detection position for optically tracking the medical instrument, in particular its position and/or orientation in space, in particular in an operating room.

Various types of medical marker elements, medical instruments and medical systems are known in the art. They are used, in particular, in operating theatres or operating rooms for determining a position and/or orientation of medical instruments, implants and parts of patients' bodies in space, in particular in the operating room. They are configured and designed to assist a surgeon, in particular when implanting implants in a patient's body.

Commonly used are marker elements in the form of a sphere forming parts of referencing units. Such referencing units can be connected to instruments. The referencing units typically comprise at least three marker elements whose position is detected with a detection device comprising a stereo camera. Such referencing units must be sufficiently stable, and therefore are usually made comprising a metal support which is rather heavy. Another disadvantage is the size of such referencing units, which often hinders a convenient use for the surgeon.

The mentioned referencing units form part of surgical navigation systems which are based on infrared technology for tracking medical instruments in the operating room. The medical instruments usually have a specific interface for coupling to the referencing unit, which is also called a navigation adapter, array or star.

Before navigation of the instrument can start, the referencing unit has to be equipped with marker elements. Usually, they are made as single-use reflective marker spheres designed for reflecting infrared radiation. A disadvantage of the state of the art systems is the time-consuming marker assembly. Typically, three to four reflective spheres have to be mounted on each referencing unit prior to the use thereof.

As the referencing unit with the reflective spheres has to be orientated toward the camara, line of sight issues occur which negatively influence a smooth and efficient workflow. Often, a further consequence is unreliable instrument tracking. In addition, as the known referencing units are bulky and heavy, they obstruct the surgical site and also change the instrument balance, having a negative effect on the handling of the medical instrument.

Therefore, it is an object of the present invention to improve a medical marker element, a medical instrument and medical system described in the outset so that they allow, in particular, an improved handling.

This object is accomplished, in accordance with the invention, in a medical marker element described at the outset in that the medical marker element comprises a sleeve surrounding a shaft portion of a shaft of the medical instrument.

A medical marker element comprising a sleeve for surrounding the shaft portion of the shaft of the medical instrument allows for an easy handling thereof. In contrast to known medical marker elements which have to be connected to a referencing unit before use, the proposed medical marker element can be easily connected to the shaft portion of the shaft of the medical instrument. It can be made small and lightweight so that there is minimal influence on the balance of the medical instrument. Further, the size of the medical marker element can be significantly reduced compared to known referencing units. Therefore, there is no negative effect when the proposed marker element is used in an operating room as the surgical site is not occluded by the referencing unit. A further advantage is the direct connection of the marker element to the instrument. This reduces the number of connections compared to referencing units known in the art which have individual connections for each marker element and an additional connection of the referencing unit and the medical instrument. Consequently, by reducing the number of connections, not only can the accuracy of the system be improved, but also the time for preparing the medical instrument by equipping the same with a single medical marker element can be significantly reduced.

It is advantageous if the marker element interface comprises a marker element axial positioning device for positioning the medical marker element in a defined axial position on a shaft portion of a shaft of the medical instrument defining a shaft longitudinal axis thereof. The marker element axial positioning device allows a defined arrangement or positioning of the medical marker element on the medical instrument. In particular, if the medical instrument has a design which is rotationally symmetrical, it is sufficient if the medical marker element is positioned on the shaft of the medical instrument in a defined axial position. For example, medical instruments in the form of palpation instruments having a rotationally symmetrical shaft with a palpation tip can be perfectly tracked in space, i.e. their position and/or orientation can be determined accurately in space, if the marker element is well-defined positioned on the shaft. As a position of the marker element in the circumferential direction with respect to the shaft longitudinal axis is not important for such instruments for determining their position and/or orientation in space, the medical marker element can have a simple design so that it can be easily handled and manufactured at low cost.

Preferably, the marker element axial positioning device comprises at least one marker element axial positioning element cooperating with an instrument axial positioning element of the medical instrument in the detection position. For example, it is possible to connect the at least one marker element axial positioning element with the instrument axial positioning element of the medical instrument in the detection position in a defined manner, so as to define a unique relative position of the marker element and the instrument. This allows an accurate positioning of the marker element on the instrument so that a position of a tip of the instrument can accurately be determined in space. Further, an orientation of the shaft of the instrument can be determined with sufficient accuracy for performing a surgical intervention with sufficient accuracy.

The medical marker element can be easily made if the at least one marker element axial positioning element extends in the circumferential direction with respect to the shaft longitudinal axis and points in a direction facing toward the shaft longitudinal axis. Such a marker element axial positioning element can easily be brought in engagement with a corresponding instrument axial positioning element which also extends in the circumferential direction with respect to the shaft longitudinal axis. This allows a positioning of the marker element relative to a tip of the instrument at a well-defined distance. If the marker element position and/or orientation is determined, the position and/or orientation of the instrument tip in space can automatically be determined by calculation if the position of the tip relative to the instrument axial positioning element of the medical instrument is known.

The medical marker element can be easily made if the marker element axial positioning element is designed in the form of a projection or a recess. Such marker element axial positioning elements can be easily brought in engagement with corresponding recesses or projections provided on the shaft of the medical instrument.

It is advantageous, if the sleeve has a sleeve wall, and if the projection is designed in the form of an annular rib protruding from an inner wall surface of the sleeve wall or if the recess is designed in the form of an annular groove in the sleeve wall. Such projections and recesses can be easily brought in engagement with corresponding recesses and projections provided on the medical instrument shaft for positioning the medical marker element in a defined manner on the medical instrument. By determining the position and/or orientation of the marker element, the medical instrument can be tracked in space in a desired manner. Any part of the medical instrument whose relative position with respect to the instrument axial positioning element is known can be determined with sufficient accuracy.

Further, it is preferred if the annular rib has a first side surface and a second side surface, if the first and second side surfaces face in opposite directions, in particular in the proximal and distal direction, and if at least one clamping element for clampingly engaging the instrument axial positioning element, in particular a corresponding recess provided on the medical instrument, is formed or arranged only on one of the first and second side surfaces. Such a design allows a well-defined engagement of the annular rib and the corresponding recess on the medical instrument. A well-defined position of the marker element on the medical instrument can be defined by the contact of the side surface of the annular rib which is not provided with the at least one clamping element and on of the first and second side surfaces. In other words, the side surface of the annular rib which is free of any clamping element defines the axial position of the marker element on the instrument shaft in connection with the recess provided on the medical instrument, in particular a corresponding surface of the recess. Therefore, the accuracy of the instrument tracking is, in particular, given by the accuracy of the position of the annular rib on the sleeve with respect to an outer shape of the medical marker element, in particular an optical pattern provided thereon, for tracking it in space. The at least one clamping element ensures a defined positioning of the side surface of the annular rib without the at least one clamping element in relation to the recess on the medical instrument.

The medical marker element can be easily manufactured if the at least one clamping element is designed in the form of a clamping projection extending away from the side surface on which it is arranged or formed. This allows, in particular, for exerting a clamping force on the medical instrument if the at least one clamping element is slightly compressed or deformed when engaging the recess on the instrument shaft. The compressed clamping element forces the side surface of the annular rib free of a clamping element against a wall of the recess on the instrument shaft so as to position the marker element in a defined manner on the instrument shaft in the axial direction. In other words, the at least one clamping element allows compensating a play between the cooperating marker element axial positioning device and the instrument axial positioning device in the detection position, which is not only inevitable but also desired in view of manufacturing tolerances.

For positioning the medical marker element also in the circumferential direction in a defined manner on the instrument shaft, it is advantageous if the at least one marker element axial positioning element comprises at least two axial positioning element sections which are spaced apart from each other in the circumferential direction. For example, a gap between two axial positioning element sections can cooperate with a corresponding projection provided on the instrument for positioning the marker element in the circumferential direction with respect to the shaft longitudinal axis on the instrument shaft in a defined manner.

In accordance with a preferred embodiment, the marker element interface comprises a marker element circumferential positioning device for positioning the medical marker element in a defined circumferential position on a shaft portion of a shaft of the medical instrument defining a shaft longitudinal axis thereof. Such a marker element interface has, in particular, the advantage that an orientation of the medical instrument can be easily determined. This is, in particular, advantageous if the medical instrument does not have a rotationally symmetrical design. For example, this is the case if the instrument shaft is at least partially curved or bears a tool element of non-rotationally symmetrical design.

It is advantageous, if the marker element circumferential positioning device comprises at least one marker element circumferential positioning element cooperating with an instrument circumferential positioning device of the medical instrument, in particular at least one instrument circumferential positioning element, in the detection position. Such a marker element circumferential positioning device allows a well-defined positioning of the medical marker element on the instrument shaft portion in the circumferential direction. A relative position of the marker element on the instrument is well-defined by the cooperating marker element circumferential positioning device and the instrument circumferential positioning device.

Preferably, the at least one marker element circumferential positioning element is designed in the form of a recess or projection on the sleeve wall for receiving a corresponding projection or recess forming an instrument circumferential positioning element of the medical instrument. Such a design allows a simple cooperation between the at least one marker element circumferential positioning element and the instrument circumferential positioning element. A accurate position of the marker element on the instrument can be easily defined by the position of the recess and projection engaging each other.

In particular, it is advantageous if the recess is open in the proximal or distal direction. This allows providing, for example, a protruding pin on the shaft which engages the recess on the marker element. The pin can protrude in the radial direction pointing away from the shaft longitudinal axis. Such a pin defines an angular position in the circumferential direction on the shaft. Of course, it is possible to have one, two, three or more marker element circumferential positioning elements which engage with one, two, three or more instrument circumferential positioning elements. It has only to be ensured, that at least one marker element circumferential positioning element engages at least one instrument circumferential positioning element in the detection position.

In accordance with a preferred embodiment, the marker element comprises at least three contact elements for contacting the shaft in the detection position. In particular, the at least three contact elements can contact the shaft in the detection position on corresponding abutment surfaces of the instrument interface. Such a design ensures a well-defined positioning of the marker element on the shaft of the medical instrument. The at least three contact elements allow providing three contact points or three contact lines for a well-defined positioning of the marker element on the instrument shaft portion. In contrast to a face contact, manufacturing tolerances can be easily compensated and the accuracy of the system significantly improved.

Preferably, the at least three contact elements are in the form of projections arranged or formed on the sleeve wall and pointing in the direction toward a marker element longitudinal axis of the marker element. This allows the at least three contact elements to contact an outer surface of the instrument shaft, in particular the instrument interface, so that the sleeve is positioned on the shaft portion in a well-defined manner. The at least three contact elements are, in particular, useful for compensating manufacturing tolerances of the sleeve.

For defining contact lines of the at least three contact elements with the instrument interface, it is preferred if the at least three contact elements extend in parallel with the marker element longitudinal axis. This allows positioning the marker element on the instrument shaft portion in a manner known as a so-called three point bearing which ensures secure and well-defined positioning of the marker element on the instrument shaft portion. In particular, the at least three contact elements can be made so as to contact flanges provided on the instrument shaft having cylindrical surfaces concentrically surrounding the shaft longitudinal axis.

In a further preferred embodiment, the marker element, in particular the sleeve, is expandable from a basic position to an expanded position, an outer diameter of the marker element being larger in the expanded position than in the basic position. Such a deformable, in particular, elastically deformable design of the marker element allows a simple connection with the shaft or a shaft portion of the medical instrument. It can be expanded from the basic position, for example, to snap onto the shaft portion. In particular, if the sleeve is provided with a lateral slot, the marker ele-ment can be laterally pushed on the shaft portion to snap thereon. This enables a quick and easy handling of a medical system.

Preferably, the marker element, in particular the sleeve, comprises a slot extending in a direction parallel or substantially parallel to a marker element longitudinal axis of the marker element. Such a design allows for a simple handling, in particular a simple connection to a shaft portion of the medical instrument. The slot can be used for simply expanding the sleeve when cooperating with the shaft portion for being snapped thereon. Preferably, a circumferential angle defined by the slot is sufficiently smaller than 180° in order to ensure that the marker element is held in the detection position securely on the shaft portion of the medical instrument. A slot having such a dimension in the circumferential direction ensures that the remaining sleeve surrounds the shaft portion over more than 180°.

In another preferred embodiment, the sleeve is completely closed. In particular, the sleeve can be completely closed in the basic position. The latter means that, although a slot is provided on the sleeve extending in parallel to the marker element longitudinal axis, the slot can be closed or substantially closed in the basic position. Thus, the sleeve completely surrounds the shaft portion of the medical instrument in the detection position. However, completely closed can also mean that the sleeve completely surrounds the shaft portion without having any slot extending in the acial direction and without the option of being expanded. Such a sleeve can be connected, for example, with the shaft by a bayonet connection or the like. However, a completely closed sleeve requires pushing it over the shaft in parallel to the shaft longitudinal axis for connecting the marker element and the instrument. An engagement from a lateral side, as is possible for a slotted sleeve as described, is not possible for a completely closed slotless sleeve.

In accordance with a further preferred embodiment, the sleeve comprises at least two sleeve portions which are arranged so as to be movable relative to each other, the at least two sleeve portions can be brought from a first position, in which they are separated by at least one slot, to a second position, in which the at least one slot is closed so that the at least two sleeve portions form a closed sleeve. Such a design can be used to form a kind of jacket which can be opened by moving the at least two sleeve portions relative to another from the second position in which the sleeve is closed, to the first position, in which the at least two sleeve portions are at least partially separated from one another by the at least one slot. This allows introducing the shaft portion into the opened sleeve and subequently closing the sleeve by moving, in particular pivoting, the at least two sleeve portions from the first position back to the second position to close the sleeve. Such a design makes it possible to simply mount the marker element on the shaft of the medical instrument.

For ensuring that the marker element cannot inadvertently fall off the shaft, the marker element comprises a locking device for locking the at least two sleeve portions in the second position. This means, in particular, that the locking device has to be unlocked for bringing the at least two sleeve portions from the second position in which the sleeve is closed, to the first position, in which the at least two sleeve portions are separated by at least one slot.

The marker element can be easily manufactured and handled if the locking device is designed in the form of a latching device comprising cooperating latching elements which are in engagement in the second position and out of engagement in the first position. In particular, a marker element can be provided which takes a basic position which is defined by the described first position in which the at least two sleeve portions are separated by at least one slot. Starting with such a marker element, a person can simply connect the medical marker element to a shaft portion of the medical instrument by introducing the shaft portion into the opened sleeve. For connecting the marker element to the shaft portion, the at least two sleeve portions have to be moved toward each other and the cooperating latching elements provided on the at least two sleeve portions, for example in the vicinity of the slot, are brought in engagement for locking the sleeve in the second position.

Moreover, in a further preferred embodiment, the medical marker element has a marker element interface which comprises a bayonet connecting element corresponding to an instrument connecting element of the instrument. A typical bayonet connecting element is formed in the form of a groove or slot having an L-shaped design. Thus, to connect the marker element to the instrument, the cooperating connecting elements of a bayonet connection have to be moved in a first direction defined by a first leg of the L-shaped bayonet connecting element and then in a second direction defined by the second leg of the bayonet connecting element. This allows both an axial and circumferential defined positioning of the marker element on the shaft of the medical instrument. In particular, the bayonet connecting element comprises the marker element axial positioning device and the marker element circumferential positioning device. For example, a leg of the bayonet connecting element can extend in the circumferential direction so as to define the marker element axial positioning device or element and the leg extending in parallel to the marker element longitudinal axis can define the marker element circumferential positioning device. Moreover, it is also conceivable that the leg of the bayonet connecting element extending in the circumferential direction has a further recess or projection cooperating with a corresponding instrument connecting element in the detection position so as to define a circumferential position of the marker element on the shaft portion of the medical instrument. In particular, such a circumferential positioning element can be arranged or formed on a spring element, in particular a leaf spring element, of the marker element limiting a groove or recess defined by the bayonet connecting element.

Further, in accordance with a preferred embodiment, the outer surface of the sleeve is provided with an optically visible pattern which is detectable by an optical detection device. Such an optical detection device can be in the form of a camera, in particular a stereo camera. The optically visible pattern can be either a randomized or regular pattern. If the pattern is known, in particular stored in a storage of a navigation system, the marker element can be easily detected by comparing the pattern of the medical marker element which is connected to the medical instrument by acquiring an image thereof with the optical detection device and comparing the acquired image with the stored pattern of the medical marker element. Depending on the optical pattern, even an orientation of the medical marker element in space can be detected by acquiring an image of the optical pattern and comparing the same with the stored pattern.

Preferably, the optical pattern comprises light and dark, in particular black and white, regions. The regions can be in the form of polygons, in particular in the form of rectangles. This allows providing an appearance of the pattern similar to a chessboard.

Preferably, the medical marker element defines a marker element longitudinal axis and the optical pattern defines border lines along light and dark regions next to each other, and the border lines extend in parallel with the marker element longitudinal axis or in a circumferential direction with respect to the marker element longitudinal axis. Such an optical pattern allows simply detecting the marker element longitudinal axis in space by detecting and extracting the border lines from an acquired image of the optical pattern of the marker element connected to an instrument.

It is advantageous if the marker element is made from one piece, in particular, it can be made monolithically. Such a marker element can be easily handled. Further, it is almost impossible that parts, in particular small pieces, of the marker element can break off and get lost in a surgical site.

In order to use the medical marker element in an operating room, it is advantageous if the marker element is made of a sterilizable plastic material. In particular, the plastic material can be sterilizable using overheated steam, beta radiation or gamma radiation. This allows sterilizing the marker element before introducing the same into an operating room.

For a simple handling of the marker element, it is preferably provided in the form of a single use marker element. If it comes sterilized and protected by a packaging to an operating room, it can be immediately used and connected to a provided medical instrument. After use, the medical marker element can be disposed of. Thus, cleaning and again sterilizing the marker element becomes superfluous.

Furthermore, the object described hereinabove is accomplished with a medical instrument described at the outset in that the instrument interface is formed on the shaft portion and designed for connecting to the marker element interface of any one of the preferred embodiments of medical marker elements described above.

Providing a medical instrument with the additional features has, in particular, the advantage that it can be easily connected to any of the medical marker elements of which preferred embodiments are described above. In particular, such an instrument allows a simple connection with a medical marker element comprising a sleeve. Depending on whether or not the sleeve is provided with a slot, the medical marker element can either be snapped on the shaft portion from a lateral side or pushed on the shaft in an axial direction defined by the shaft and/or the shaft portion.

Preferably, the medical instrument interface and the marker element interface form a connecting device for the at least one of force locking and form locking connection of the medical instrument and the medical marker element in the detection position. Such a connecting device allows to connect the medical marker element and the medical instrument in a simple and quick manner. Moreover, such a connecting device ensures, in particular, a secure connection of the marker element on the instrument. Further, such a connecting device can define a single, i.e. unique, detection position, i.e. there is only one option to connect the medical marker element to the medical instrument. Thus, a medical system for tracking the medical marker element in space can accurately determine a position of a part of the medical instrument, for example a tip thereof.

The marker element and the medical instrument can be easily and quickly connected if the connecting device is in the form of a snap-fit or a bayonet connecting device.

It is advantageous, if the instrument interface comprises an instrument axial positioning element which cooperates with the marker element axial positioning element in the detection position for positioning the medical marker element on the medical instrument in a defined axial position on the shaft portion. The instrument axial positioning element allows the positioning of the marker element on the instrument in a well-defined axial position. In particular, if the instrument or the instrument shaft are rotationally symmetric, a relative position of an instrument portion, for example a tip thereof, can be accurately determined if the position of the marker element is accurately determined.

Preferably, the instrument axial positioning element extends in the circumferential direction with respect to the shaft longitudinal axis and points in a direction facing away from the shaft longitudinal axis. Such a design allows, in particular, the positioning of the marker element in a defined axial position on the shaft of the instrument. In particular, such an instrument axial positioning element can prevent a movement of the marker element in the axial direction. However, depending on its design, a rotation of the medical marker element relative to the shaft longitudinal axis, namely thereabout, can still be possible.

The instrument interface can be manufactured and constructed in a simple and cost effective manner if the instrument axial positioning element is designed in the form of a recess or a projection on the shaft portion. Such a design allows to bring the instrument axial positioning element in engagement with a corresponding projection or a recess provided on the medical marker element.

A quick and simple connection between the marker element and the instrument is made possible if the recess defines an annular groove. A further advantage of such a design is that no projections are necessary on the instrument shaft, which could cause harm to a user if the instrument is not connected to a marker element.

In accordance with a further preferred embodiment, the instrument axial positioning element has a first width extending in the axial direction, the marker element positioning element has second width extending in the axial direction, the at least one clamping element has a third width extending in the axial direction, and the first width is smaller than the sum of the second and third widths. Such dimensions of the cooperating elements of the instruments and the marker element ensure that the marker element is positioned well-defined on the instrument shaft in the detection position. In particular, the at least one clamping element with its third width can provide the required clamping action for positioning the marker element on the shaft so as to be well-defined in the axial direction.

For improving the handling of the medical instrument and the medical marker element when connecting them, it is preferred if the instrument interface comprises a spreading device for spreading the sleeve of the medical marker element when connecting to the shaft. Such a spreading device preferably cooperates with a marker element having a sleeve provided with a slot extending in the axial direction. The spreading device results in a spreading action when cooperating with the sleeve of the medical marker element so that the latter can easily snap on the shaft of the medical instrument.

A marker element with a slotted sleeve can be easily spread if the spreading device comprises two first flat surfaces which are formed or arranged on the shaft so as to define a spreading angle between them. Such a design allows a user to bring the slotted sleeve in contact with the two first flat surfaces and then to push the marker element laterally against the shaft of the instrument. Such a movement results in a spreading action when moving the marker element further toward the shaft. In other words, the spreading device automatically spreads the sleeve comprised by the marker element when it is pushed on the shaft.

The design of the medical instrument can be made particularly simple if the two first flat surfaces define first flat planes and if the two first flat planes intersect in a straight line which extends in parallel or substantially in parallel with the shaft longitudinal axis. Such a design simply supports a defined axial positioning of the marker element on the shaft as it supports orientating the marker element so as to be aligned in parallel with the shaft longitudinal axis.

The sleeve of the marker element can be easily spread if the spreading angle is in a range from about 1° to about 30°. In particular, the spreading angle is about 15°.

In order to avoid a sharp edge being formed on the medical instrument, it is preferred that the two first flat surfaces are formed or arranged separate from each other on the shaft portion. In particular, the two first flat surfaces can be connected by a flat surface so that each of the two first flat surfaces and the connecting surface form an obtuse angle with respect to each other. Such a design reduces potential harm to a user.

It is advantageous, if the instrument interface comprises two second flat surfaces which are formed or arranged on the shaft, if the two second flat surfaces extend in parallel to one another and each of the two second flat surfaces adjoins one of the two first flat surfaces so that the first and second flat surfaces adjoining one another define a common edge on the shaft, and if the common edge extends in parallel with the shaft longitudinal axis. The two second flat surfaces have, in particular, the advantage that the marker element is maintained in a spread position while sliding over the second flat surfaces. Further, the snap-on effect is less strong if the second flat surfaces are provided, compared to a spreading device having only the two flat surfaces defining the spreading angle between them.

Further, it is advantageous if the medical instrument interface comprises a proximal flange and a distal flange arranged or formed on the shaft portion, and if the proximal and distal flanges define abutment surfaces pointing in radial direction for the contacting elements of the marker element. Providing such flanges ensures, in particular, a well-defined positioning of the marker element on the shaft. In particular, a so-called three point bearing can be made possible which takes place only between the proximal and distal flanges and the contact elements of the marker elements.

For a well-defined positioning of the medical marker element on the shaft of the instrument, it is preferred if the abutment surfaces are in the form of cylindrical surfaces or portions of cylindrical surfaces and if the cylindrical surfaces are concentric with respect to the shaft longitudinal axis. Such abutment surfaces ensure that the sleeve of the marker element is positioned concentrically with respect to the shaft longitudinal axis.

In order to avoid that the medical marker element adopts an axial position on the shaft that is not desired, it is advantageous if the spreading device is arranged between the proximal flange and the distal flange. Such a design supports a user to connect the marker element and the shaft in the correct axial position. Further, it allows to guide the marker element such that the marker element axial positioning element and the instrument axial positioning element can engage easily when connecting them.

The instrument interface can be made as small as possible if the instrument axial positioning element is arranged between the proximal flange and the distal flange. Thus, the proximal and distal flanges can limit the instrument interface in the axial direction on both sides.

In accordance with a further preferred embodiment, the instrument interface comprises an instrument circumferential positioning device cooperating with the marker element circumferential positioning device in the detection position. Such an instrument circumferential positioning device ensures that the marker element takes the desired angular position in the circumferential direction in the detection position.

It is advantageous, if the instrument circumferential positioning device comprises at least one instrument circumferential positioning element which is designed in the form of a projection or recess pointing in a radial direction with respect to the shaft longitudinal axis. In particular, the at least one instrument circumferential positioning element points away from the shaft longitudinal axis. The at least one instrument circumferential positioning element can define an angular position relative to the shaft of the instrument so that the marker element can be positioned on the shaft in a well-defined manner in the circumferential direction. This is, in particular, helpful if the instrument does not have a rotational symmetry. For example, if the shaft is at least partially curved, a tip of the shaft can only be determined if not only the actual position of the medical marker element on the instrument shaft is known but also the position of the marker element in the circumferential direction with respect to the shaft longitudinal axis.

The instrument interface can be made particularly simple if the projection is designed in the form of the positioning pin. Such a pin, in particular pointing in the radial direction with respect to the shaft longitudinal axis, can easily cooperate with a corresponding recess on the marker element. Further, such a design also makes it possible that the marker element can be connected to the instrument shaft in only a single and unique orientation with respect to the shaft longitudinal axis.

In accordance with a further preferred embodiment, the connecting device, in particular the bayonet connecting device, comprises the at least one instrument circumferential positioning element. In particular, a pin pointing in the radial direction away from the shaft longitudinal axis can cooperate with a bayonet connecting element being L- or U-shaped for connecting the marker element with the shaft taking advantage of a bayonet connection.

Further, it is preferred if the medical instrument has an instrument tip, in particular a palpation tip, defining a distal end of the medical instrument, and if the instrument tip lies on the shaft longitudinal axis. Such a medical instrument can be made in particular with a shaft having a rotational symmetry. Therefore, such a medical instrument only requires a defined axial positioning of the marker element on the shaft in order to determine the position of the instrument tip in space based on the tracked marker element.

Furthermore, the object described hereinabove is accomplished with a medical system described at the outset in that the medical instrument is in the form of a medical instrument in accordance with any one of the preferred embodiments of medical instruments described above, and in that the at least one medical marker element is in the form of a medical marker element in accordance with any one of the preferred embodiments of medical marker elements described above.

A medical instrument comprising at least one medical instrument in the form of a medical instrument according to any one of the preferred embodiments described above and comprising a medical marker element in accordance with any one of the preferred embodiments described above has, in particular, the advantages described above in connection with the preferred embodiments of medical instruments and the preferred embodiments of medical marker elements. In other words, such a medical system allows to track medical instruments in space with high accuracy on the one hand and at low cost on the other hand.

The medical instrument and, in particular, the medical marker element can be easily and accurately tracked in space, in particular in an operating room, if the medical system comprises an optical detection device for optically detecting the marker element, and if the optical detection device is designed for determining at least one of a position and orientation of the medical marker element in space, in particular in an operating room. Such a medical system has, in particular, the advantage that the tracking of the instrument is made possible by tracking the marker element using at least one optical camera. Distance measuring devices are not required. The position and orientation in space can be easily determined by capturing images of the marker element in space, in particular in the operating room.

The above description comprises, in particular, the following numbered embodiments of medical marker elements, medical instrument and medical systems:
1. Medical marker element (56) which is formed for the releasable connection to a medical instrument (46), the medical marker element (56) being optically detectable for determining a position and/or orientation of the medical marker element (56) in space, in particular in an operating room, wherein the medical marker element (56) comprises a marker element interface (58) which is formed for the connection to a corresponding instrument interface (54) of the medical instrument (46) in a detection position, wherein the marker element interface (58) and the instrument interface (54) are in engagement in the detection position in a defined manner and are out of engagement in a cleaning position, characterized in that the medical marker element (56) comprises a sleeve (112) surrounding a shaft portion (50) of a shaft (48) of the medical instrument (22).
2. Medical marker element in accordance with embodiment 1, characterized in that the marker element interface (58) comprises a marker element axial positioning device (156) for positioning the medical marker element (56) in a defined axial position on a shaft portion (50) of a shaft (48) of the medical instrument (46) defining a shaft longitudinal axis (52) thereof.
3. Medical marker element in accordance with embodiment 2, characterized in that the marker element axial positioning device (156) comprises at least one marker element axial positioning element (158) cooperating with an instrument axial positioning element (68) of the medical instrument (46) in the detection position.
4. Medical marker element in accordance with embodiment 3, characterized in that the at least one marker element axial positioning element (158) extends in the circumferential direction with respect to the shaft longitudinal axis (52) and points in a direction facing toward the shaft longitudinal axis (52).
5. Medical marker element in accordance with embodiment 3 or 4, characterized in that the marker element axial positioning element (158) is designed in the form of a projection (160) or a recess.
6. Medical marker element in accordance with embodiment 5, characterized in that the sleeve (112) has a sleeve wall (124), and in that the projection (160) is designed in the form of an annular rib (162) protruding from an inner wall surface (164) of the sleeve wall (124) or in that the recess is designed in the form of an annular groove in the sleeve wall (124).
7. Medical marker element in accordance with embodiment 6, characterized in that the annular rib (162) has a first side surface (174) and a second side (176) surface, in that the first and second side surfaces face (174, 176) in opposite directions, in particular in the proximal and distal direction, and in that at least one clamping element (180) for clampingly engaging the instrument axial positioning element (68), in particular a corresponding recess provided on the medical instrument, is formed or arranged only on one of the first and second side surfaces (174, 176).
8. Medical marker element in accordance with embodiment 7, characterized in that the at least one clamping element (180) is designed in the form of a clamping projection (188) extending away from the side surface (174) on which it is arranged or formed.
9. Medical marker element in accordance with any one of embodiments 3 to 8, characterized in that the at least one marker element axial positioning element (158) comprises at least two axial positioning element sections (192) which are spaced apart from each other in the circumferential direction.
10. Medical marker element in accordance with any one of the preceding embodiments, characterized in that the marker element interface (58) comprises a marker element circumferential positioning device (194) for positioning the medical marker element (46) in a defined circumferential position on a shaft portion (50) of a shaft (48) of the medical instrument (46) defining a shaft longitudinal axis (52) thereof.
11. Medical marker element in accordance with embodiment 10, characterized in that the marker element circumferential positioning device (194) comprises at least one marker element circumferential positioning element (196) cooperating with an instrument circumferential positioning device (104) of the medical instrument (46), in particular at least one instrument circumferential positioning element (106), in the detection position.
12. Medical marker element in accordance with embodiment 11, characterized in that the at least one marker element circumferential positioning element (196) is designed in the form of a recess (198) or a projection on the sleeve wall (124) for receiving a corresponding projection (108) or recess forming an instrument circumferential positioning element (106) of the medical instrument (46).
13. Medical marker element in accordance with embodiment 12, characterized in that the recess (198) is open in the proximal or distal direction.
14. Medical marker element in accordance with any one of the preceding embodiments, characterized in that the marker element (56) comprises at least three contact elements (204, 206) for contacting the shaft (48) in the detection position, in particular on corresponding abutment surfaces (100, 102) of the instrument interface (54).
15. Medical marker element in accordance with embodiment 14, characterized in that the at least three contact elements (204, 206) are in the form of projections (208, 210) arranged or formed on the sleeve wall (124) and pointing in the direction toward a marker element longitudinal axis (116) of the marker element (56).
16. Medical marker element in accordance with embodiment 14 or 15, characterized in that the at least three contact elements (204, 206) extend in parallel with the marker element longitudinal axis (116).
17. Medical marker element in accordance with any one of the preceding embodiments, characterized in that the marker element (56), in particular the sleeve (112), is expandable from a basic position to an expanded position, an outer diameter of the marker element (56) being larger in the expanded position than in the basic position.
18. Medical marker element in accordance with any one of the preceding embodiments, characterized in that the marker element (56), in particular the sleeve (112), comprises a slot (114) extending in a direction parallel or substantially parallel to a marker element longitudinal axis (116) of the marker element (56).
19. Medical marker element in accordance with any one of the preceding embodiments, characterized in that the sleeve (112) is completely closed, in particular in the basic position.
20. Medical marker element in accordance with any one of the preceding embodiments, characterized in that the sleeve (112) comprises at least two sleeve portions (238, 240) which are arranged so as to be movable relative to each other, in that the at least two sleeve portions (238, 240) can be brought from a first position, in which they are separated by at least one slot (262), to a second position, in which the at least one slot (262) is closed so that the at least two sleeve portions (238, 240) form a closed sleeve (112).
21. Medical marker element in accordance with embodiment 20, characterized in that the marker element comprises a locking device (264) for locking the at least two sleeve portions (238, 240) in the second position.
22. Medical marker element in accordance with embodiment 21, characterized in that the locking device (264) is designed in the form of a latching device (266) comprising cooperating latching elements (268, 270) which are in engagement in the second position and out of engagement in the first position.
23. Medical marker element in accordance with any one of the preceding embodiments, characterized in that the marker element interface (58) comprises a bayonet connecting element (222) corresponding to an instrument connecting element of the instrument (46), wherein, in particular, the bayonet connecting element (222) comprises the marker element axial positioning device (156) and the marker element circumferential positioning device (194).
24. Medical marker element in accordance with any one of the preceding embodiments, characterized in that the outer surface (144) of the sleeve (112) is provided with an optically visible pattern (146) which is detectable by an optical detection device (28), in particular a camera (32).
25. Medical marker element in accordance with embodiment 24, characterized in that the optical pattern (144) comprises light and dark, in particular black and white, regions (148, 150), in particular in the form of polygons, further in particular in the form of rectangles.
26. Medical marker element in accordance with embodiment 24 or 25, characterized in that the medical marker element (56) defines a marker element longitudinal axis (116) and in that the optical pattern (146) defines border lines (152, 154) along light and dark regions (148, 150) next to each other, and in that the border lines (152) extend in parallel with the marker element longitudinal axis (116) or in a circumferential direction with respect to the marker element longitudinal axis (116).
27. Medical marker element in accordance with any one of the preceding embodiments, characterized in that the marker element (56) is made from one piece, in particular monolithically.
28. Medical marker element in accordance with any one of the preceding embodiments, characterized in that the marker element (56) is made of a sterilizable plastic material.
29. Medical marker element in accordance with any one of the preceding embodiments, characterized in that the medical marker element (56) is in the form of a single use medical marker element (56).
30. Medical instrument (46) comprising a shaft (48), wherein the shaft (48) has a shaft portion (50), which defines a shaft longitudinal axis (52) of the instrument (46), and wherein the medical instrument (46) has an instrument interface (54) for releasably connecting to a medical marker element interface (58) of a medical marker element (56), which is optically detectable for detecting a position and/or orientation of the medical marker element (56) and the medical instrument (46) connected thereto in space, in particular in an operating room, wherein the instrument interface (54) and the marker element interface (58) are in engagement in a detection position in a defined manner and are out of engagement in a cleaning position, characterized in that the instrument interface (54) is formed on the shaft portion (50) and is designed for connecting to the marker element interface (58) of a medical marker element (56) in accordance with any one of the preceding embodiments.
31. Medical instrument in accordance with embodiment 30, characterized in that the medical instrument interface (54) and the marker element interface (58) form a connecting device (64) for the at least one of force locking and form locking connection of the medical instrument (46) and the medical marker (56) element in the detection position.
32. Medical instrument in accordance with embodiment 31, characterized in that connecting device (64) is in the form of a snap-fit or bayonet connecting device (66, 222).
33. Medical instrument in accordance with any one of embodiments 30 to 32, characterized in that the instrument interface (54) comprises an instrument axial positioning element (68) which cooperates with the marker element axial positioning element (156) in the detection position for positioning the medical marker element (56) on the medical instrument (46) in a defined axial position on the shaft portion (50).
34. Medical instrument in accordance with embodiment 33, characterized in that the instrument axial positioning element (68) extends in the circumferential direction with respect to the shaft longitudinal axis (52) and points in a direction facing away from the shaft longitudinal axis (52).
35. Medical instrument in accordance with embodiment 33 or 34, characterized in that the instrument axial positioning element (68) is designed in the form of a recess (70) or a projection on the shaft portion (50).
36. Medical instrument in accordance with embodiment 35, characterized in that the recess (70) defines an annular groove (72).
37. Medical instrument in accordance with any one of embodiments 33 to 36, characterized in that the instrument axial positioning element (68) has a first width (74) extending in the axial direction, in that the marker element axial positioning element (158) has a second width (178) extending in the axial direction, in that the at least one clamping element (180) has a third width (182) extending in the axial direction, and in that the first width (74) is smaller than the sum of the second and third widths (178, 182).
38. Medical instrument in accordance with any one of embodiments 30 to 37, characterized in that the instrument interface (54) comprises a spreading device (76) for spreading the sleeve of the medical marker element when connecting to the shaft.
39. Medical instrument in accordance with embodiment 38, characterized in that the spreading device (76) comprises two first flat surfaces (78) which are formed or arranged on the shaft (48) so as to define a spreading angle (80) between them.
40. Medical instrument in accordance with embodiment 39, characterized in that the two first flat surfaces (78) define first flat planes (82) and that the two first flat planes (82) intersect in a straight line (84) which extends in parallel or substantially in parallel with the shaft longitudinal axis (52).
41. Medical instrument in accordance with embodiment 39 or 40, characterized in that the spreading angle (80) is in a range from about 1° to about 30°, in particular the spreading angle (80) is about 15°.
42. Medical instrument in accordance with any one of embodiments 39 to 41, characterized in that the two first flat surfaces (82) are formed or arranged separate from each other on the shaft portion (50).
43. Medical instrument in accordance with any one of embodiments 39 to 42, characterized in that the instrument interface (54) comprises two second flat surfaces (88) which are formed or arranged on the shaft (48), in that the two second flat surfaces (88) extend in parallel to one another, in that each of the two second flat surfaces (88) adjoins one of the two first flat surfaces (78) so that the first and second flat surfaces (78, 88) adjoining one another define a common edge (90) on the shaft (48) and in that the common edge (90) extends in parallel with the shaft longitudinal axis (52).
44. Medical instrument in accordance with any one of embodiments 30 to 43, characterized in that the medical instrument interface (54) comprises a proximal flange (96) and a distal flange (98) arranged or formed on the shaft portion (50), and in that the proximal and distal flanges (96, 98) define abutment surfaces pointing in radial direction for the contact elements (204, 206) of the marker element (56).
45. Medical instrument in accordance with embodiment 44, characterized in that the abutment surfaces (100, 102) are in the form of cylindrical surfaces or portions of cylindrical surfaces, and in that the cylindrical surfaces are concentric with respect to the shaft longitudinal axis (52).
46. Medical instrument in accordance with embodiment 44 or 45, characterized in that spreading device (76) is arranged between the proximal flange (96) and the distal flange (98).
47. Medical instrument in accordance with any one of embodiments 44 to 46, characterized in that instrument axial positioning element (68) is arranged between the proximal flange (96) and the distal flange (98).
48. Medical instrument in accordance with any one of embodiments 30 to 47, characterized in that the instrument interface (54) comprises an instrument circumferential positioning device (104) cooperating with the marker element circumferential positioning device (194) in the detection position.
49. Medical instrument in accordance with embodiment 48, characterized in that the instrument circumferential positioning device (104) comprises at least one instrument circumferential positioning element (166) which is designed in the form of a projection (108) or recess pointing in a radial direction with respect to the shaft longitudinal axis (52), in particular pointing away from the shaft longitudinal axis (52).
50. Medical instrument in accordance with embodiment 49, characterized in that the projection (108) is designed in the form of a positioning pin (110).
51. Medical instrument in accordance with any one of embodiments 31 to 50, characterized in that the connecting device (64), in particular the bayonet connecting device (222), comprises the at least one instrument circumferential positioning element (106).
52. Medical instrument in accordance with any one of embodiments 30 to 51, characterized in that the medical instrument (46) has an instrument tip (60), in particular a palpation tip, defining a distal end (62) of the medical instrument (46), and in that the instrument tip (60) lies on the shaft longitudinal axis (52).
53. Medical system (10) comprising at least one medical instrument (46) and at least one medical marker element (56), the at least one marker element (56) being releasably connectable to the at least one medical instrument (46) in a detection position for optically tracking the medical instrument (46), in particular its position and/or orientation in space, in particular in an operating room, characterized in that the medical instrument (46) is in the form of a medical instrument (46) in accordance with any one of embodiments 30 to 52 and in that the at least one medical marker element (56) is in the form of a medical marker element (56) in accordance with any one of embodiments 1 to 29.
54. Medical system (10) in accordance with embodiment 53, characterized in that the medical system (10) comprises an optical detection device (28) for optically detecting the marker element, and in that the optical detection device (28) is designed for determining at least one of a position and orientation of the medical marker element ((56) in space, in particular in an operating room.

The subsequent description of preferred embodiments of the invention serves in conjunction with the illustration for further explanation. In the drawings:
- Figure 1:: shows a schematic depiction of a medical navigation system according to the prior art;
- Figure 2:: shows a lateral side view of a medical instrument bearing a medical marker element;
- Figure 3:: shows a perspective view of a marker element and a shaft portion of the medical instrument before connecting the same in a cleaning position;
- Figure 4:: shows a similar depiction as Figure 3 but in the detection position when the marker element is connected to the shaft of the medical instrument;
- Figure 5:: shows a perspective view of the marker element;
- Figure 6:: shows a further perspective, partially broken view of the marker element shown in Figure 5 in the direction of arrow A in Figure 5;
- Figure 7:: shows a side view of the marker element;
- Figure 8:: shows a view in the direction of arrow B in Figure 7;
- Figure 9:: shows a further side view of the marker element shown in Figure 7;
- Figure 10:: shows a view in the direction of arrow C in Figure 9;
- Figure 11:: shows a partial view of the arrangement in Figure 2 with a sectional view the marker element taken along line 11-11 in Figure;
- Figure 12:: shows an enlarged view of area D in Figure 11;
- Figure 13:: shows a sectional view taken along line 13-13 in Figure 11 while connecting the medical marker element and the medical instrument;
- Figure 14:: shows a sectional view taken along lines 13-13 in the detection position;
- Figure 15:: shows a sectional view of the arrangement shown in Figure 11 along line 15-15;
- Figure 16:: shows a perspective side view of a further embodiment of a medical marker element while connecting to a medical instrument;
- Figure 17:: shows a depiction similar to Figure 16 with the marker element and the medical instrument in the detection position;

- Figure 18:: shows a perspective partial view of a further embodiment of medical marker element while connecting to a further embodiment of a medical instrument; and
- Figure 19:: shows a view similar to Figure 18 with the medical marker element in the detection position.

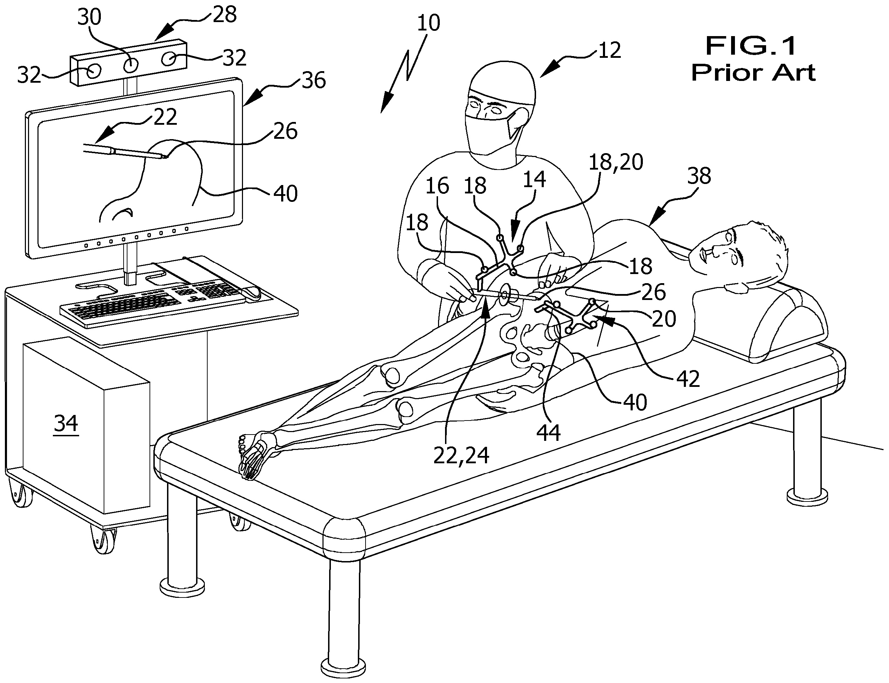

Figure 1 shows a medical system 10 used in an operating room for assisting a surgeon 12 in connection with the implantation of artificial joints. The system 10 shown in Figure 1 defines well-known prior art.

The medical system 10 comprises commonly used referencing units 14 having a cross-shaped carrier element 16 on which four marker elements 18 are arranged in a well-defined spatial relationship. The marker elements 18 are in the form of reflective spheres, in particular highly reflective for infrared radiation.

The referencing unit 14 is releasably connectable to a medical instrument 22, for example a palpation instrument 24 having a tip 26. The referencing unit 14 is arranged on the medical instrument 22 in a well-defined manner, in particular in a well-defined spatial relationship with respect to the tip 26. As the arrangement of the referencing unit 14 with respect to the tip 26 is known, tracking the referencing unit 14 in the operating room allows tracking the tip 26, in particular a position and/or orientation thereof in the operating room, i.e. in space.

The medical system 10 comprises an optical detection unit 28 comprising a radiation source 30 and two cameras 32 arranged separate from one another for defining a stereo camera arrangement.

The position of the referencing unit 14 in the operating room can be determined by acquiring pictures with the two cameras 32 of the optical detection unit 28 which is designed to detect electromagnetic radiation reflected by the marker elements 18. By means of the two cameras 32, stereo images can be captured.

The position and/or orientation of the referencing unit 14 in the operating room can be determined with high accuracy by means of a computer processing of the image data acquired by the optical detection unit 28 which is in a cooperative relationship with the computer 34. A display 36 is used for displaying the tip 26 of the medical instrument 22, in particular, relative to a patient's bone, for example a hip 40 of the patient 38. The position of the medical instrument 22 is determined in a reference coordinate system defined by a further referencing unit 42 attached to the patient's bone using a bone fastener 44, for example a bone screw or a bone pin.

As can be seen in Figure 1, the design of the referencing unit 14 has significant disadvantages. The carrier element 16 is quite large. As it has to be sufficiently stable, it is typically made of a metal. Consequently, the referencing unit 14 has a significant weight, which influences the balance of the medical instrument 22 to which the referencing unit 14 is attached. A further disadvantage is the size of the referencing unit 14. As the four marker elements 18 have to be arranged with sufficient space from each other for distinguishing them in images captured by the optical detection unit 28, such known referencing units 14 hamper the surgeon's 12 view of the surgical site.

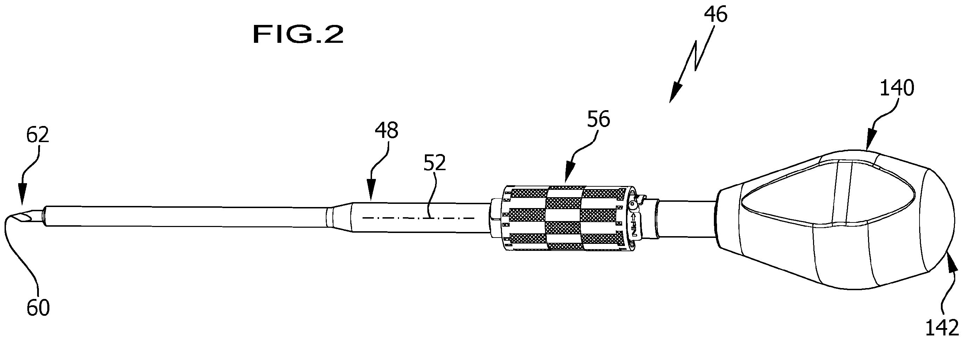

In order to overcome these disadvantages, an improved medical instrument 46 is proposed. An exemplary embodiment of such a medical instrument 46 is shown in Figure 2.

The medical instrument 46 comprises a shaft 48. The shaft 48 has a shaft portion 50, which defines a shaft longitudinal axis 52 of the instrument 46.

Further, the medical instrument 46 has an instrument interface 54. The instrument interface 54 is designed for being releasably connectable to a medical marker element 56. The medical marker element 56 is optically detectable for determining a position and/or orientation of the medical marker element 56 and the medical instrument 46 connected thereto in space, in particular in an operating room.

The medical marker element 56 comprises a marker element interface 58 which is formed for the connection to the instrument interface 54 of the medical instrument 46 in a detection position. An example of a detection position is shown in Figure 2.

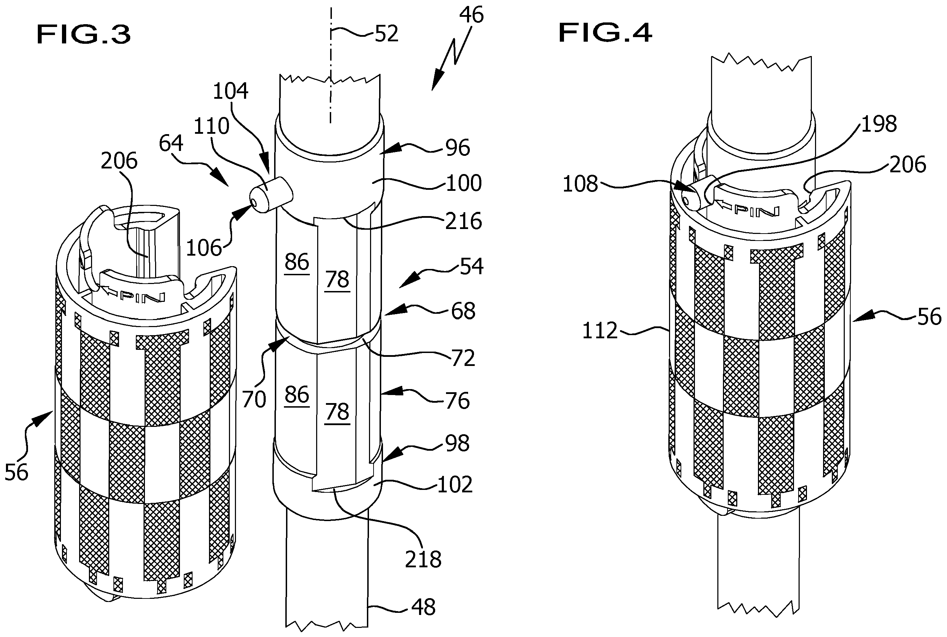

The marker element interface 58 and the instrument interface 54 are in engagement in the detection position in a defined manner. They are out of engagement in a cleaning position which is, for example, shown in Figure 3.

The instrument interface 54 is formed on the shaft portion 50 and is designed for connecting to the marker element interface 58 of the medical marker element 56.

The medical instrument 46 has an instrument tip 60, which can be used as a palpation tip. The instrument tip 60 defines a distal end 62 both of the shaft 48 and the medical instrument 46. Further, the instrument tip 60 of the embodiment of the medical instrument 46 shown in Figure 2 lies on the shaft longitudinal axis 52.

The medical instrument 46 can be used for replacing the medical instrument 22, i.e. instead the latter. This means that the remaining components and elements of the medical system 10 described above in connection with Figure 1 can also be used in cooperation with the medical instrument 46. In other words, the medical system 10 comprises at least one medical instrument 46 and at least one medical marker element 56.

The at least one marker element 56 is releasably connectable to the at least one medical instrument 46 in the detection position for optically tracking the medical instrument 46. In particular, the medical system 10 can determine the position and/or orientation of the medical instrument 46 in space, in particular in the operating room.

Moreover, the optical detection unit 28 can be used for determining at least one of a position and an orientation of the medical marker element 46 in space, i.e. in particular in the operating room.

The medical instrument interface 54 and the marker element interface 58 form a connecting device 64 for the at least one of force locking and form locking connection of the medical instrument 46 and the medical marker element 46 in the detection position.

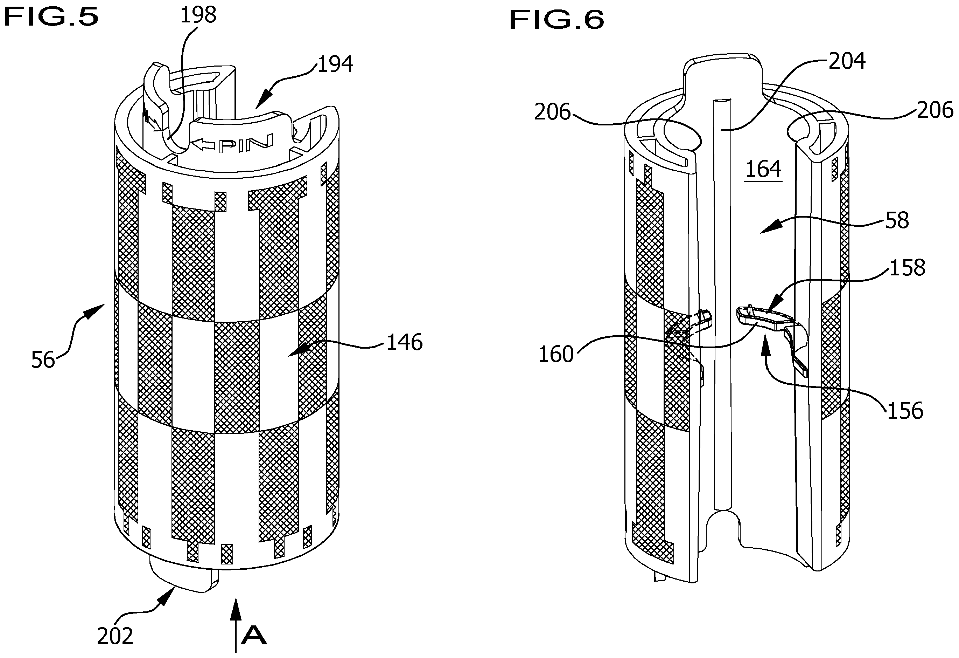

The embodiment of the connecting device 64 shown in Figures 1 to 15 is in the form a snap-fit connecting device 66.

The instrument interface 54 comprises an instrument axial positioning element 68. The instrument axial positioning element 68 extends in the circumferential direction with respect to the shaft longitudinal axis 52 and points in the direction facing away from the shaft longitudinal axis 52. The instrument axial positioning element 68 is designed in the form of a recess 70 on the shaft portion 50.

Alternative embodiments not shown in the drawings can have an instrument axial positioning element 68 in the form of a projection formed on the shaft portion 50.

The recess 70 of the embodiment shown in Figures 1 to 15 defines an annular groove 72.

The instrument axial positioning element 68 has a first width 74 extending in an axial direction defined by the shaft longitudinal axis 52.

Further, the instrument interface 54 comprises a spreading device 76. The spreading device 76 comprises two first flat surfaces 78 which are formed or arranged on the shaft 48 so as to define a spreading angle 80 between them. The two first flat surfaces 78 define a so-called "dihedron".

The two first flat surfaces 78 define first flat planes 82. The two first flat planes 82 intersect in a straight line 84 which extends in parallel with the shaft longitudinal axis 52.

The spreading angle 80 is in a range from about 1° to about 30°. The spreading angle 80 of the embodiment shown in the drawings has a value of about 15°.

The two first flat surfaces 78 are formed or arranged separate from each other on the shaft portion 50. They are connected by a portion 86 of an outer cylindrical surface of the shaft 48.

The embodiment of the medical instrument 46 shown in Figures 1 to 15 has an instrument interface 54 which comprises two second flat surfaces 88 which are also formed or arranged on the shaft 48. The two second flat surfaces 88 extend in parallel to one another. Further, the two second flat surfaces 88 each adjoin one of the two first flat surfaces 78, so that the first and second flat surfaces 78 and 88 adjoining one another define a common edge 90 on the shaft 48. The two common edges 90 defined by the respective pairs of first and second flat surfaces 78, 88 extend in parallel with the shaft longitudinal axis 52.

The second flat surfaces 88 form two or more edges 92 with a further outer cylindrical surface portion 94 pointing in the opposite direction with respect to the outer cylindrical surface portion 86.

The medical instrument interface 54 comprises a proximal flange 96 and a distal flange 98 which are arranged or formed on the shaft portion 50.

The proximal and distal flanges 96 and 98 define abutment surfaces 100 and 102 having a substantially cylindrical shape. This means that the two abutment surfaces 100 and 102 are in the form of cylindrical surfaces or portions of cylindrical surfaces. The cylindrical surfaces are concentric with respect to the shaft longitudinal axis 52.

As can be best seen in Figure 3, the spreading device 76 is arranged between the proximal flange 96 and the distal flange 98.

Moreover, the instrument axial positioning element 68 is arranged between the proximal flange 96 and the distal flange 98.

The instrument interface 54 comprises an instrument circumferential positioning device 104. The instrument circumferential positioning device 104 comprises at least one instrument circumferential positioning element 106. The instrument circumferential positioning element 106 is designed in the form of a projection 108 pointing in radial direction with respect to the shaft longitudinal axis 52. It points away from the shaft longitudinal axis 52 as shown, in particular, in Figure 3.

The instrument circumferential positioning element 106 can be in the form of a recess in alternative embodiments not shown in the drawings.

As shown in Figures 3 and 4, the projection 108 is designed in the form of a positioning pin 110.

Figures 1 to 15 depict a first embodiment of a medical marker element 56. It is designed to be releasably connectable to the embodiment of the medical instrument 46 described above. For connecting the medical marker element 56 to the medical instrument 46, the medical marker element 56 comprises a marker element interface 58.

Further, the medical marker element 56 comprises a sleeve 112 for surrounding the shaft portion 50 of the shaft 48 of the medical instrument at least partially.

The sleeve 112 is of slotted design, i.e. the sleeve 112 comprises a slot 114 extending in a direction parallel to a marker element longitudinal axis 116 of the marker element 56. The slot 114 defines a slot angle 118 extending over an angular range of about 90° in the circumferential direction with respect to the marker element longitudinal axis 116. As the slot 114 defines the slot angle 118, the sleeve 112 surrounds the shaft portion 50 in the detection position over an angular range defined by a sleeve angle 120 of about 270° which complements the slot angle 118 to 360°.

The slot 114 of the sleeve 112 defines a lateral opening 122 through which the shaft portion 50 can be introduced for connecting the medical instrument 46 and the medical marker element 56 in the detection position.

The sleeve 112 defines a sleeve wall 124 which has a wall thickness 126 in the radial direction with respect to the marker element longitudinal axis 116.

It should be noted that the marker element longitudinal axis 116 and the shaft longitudinal axis 52 coincide when the marker element 56 and the medical instrument 56 adapt the detection position.

The sleeve wall 124 is formed concentrically with respect to the marker element longitudinal axis 116.

The sleeve 112 comprises several wall openings 128, which are formed in the sleeve wall 124 and extend concentrically with respect to the marker element longitudinal axis 116 through the sleeve wall 124. Wall openings 128 are separated by webs 130. In other words, the sleeve wall 124 comprises an outer sleeve wall 132 and an inner sleeve wall 134, which are separated by the wall openings 128. The webs 130 connect the inner and outer sleeve walls 132 and 134.

The wall openings 128 extend through the sleeve wall 124 in the axial direction and are open both in the distal and proximal direction.

The sleeve 112 defines a proximal end face 136 and a distal end face 138. The proximal end face 136 faces in the proximal direction, the distal end face 138 faces in the distal direction. The distal direction of the medical instrument 46 is defined as the direction facing the instrument tip 60. On the other hand, the proximal direction is the direction facing a handle 140 arranged on the shaft 48 and defining a proximal end 142 of the medical instrument 46.

The sleeve 112 has an outer surface 144 which is defined by an outer surface of the outer sleeve wall 132. The outer surface 144 is provided with an optically visible pattern 146 which is detectable by the optical detection unit 28, in particular by the cameras 32 comprised by the optical detection unit 28. The optical pattern 144 comprises light and dark regions 148 and 150 in the form of rectangles. The light and dark regions 148 and 150 may be colored in white and black. The light and dark regions 148, 150 form surfaces areas of the outer surface 144.

Further, the optical pattern 146 defines border lines along light and dark regions 148, 150 adjoining each other. The border lines 152 extend in parallel with the marker element longitudinal axis 116. The border lines 154 extend perpendicularly to the border lines 152, namely in the circumferential direction with respect to the marker element longitudinal axis 116.

The marker element interface 58 is designed to connect to the instrument interface 54 in a form- and positive-locking manner so that the marker element 56 takes a well-defined position on the shaft portion 50 in the axial direction.

The marker element interface 58 comprises a marker element axial positioning device 156.The marker element axial positioning device 156 comprises a marker element axial positioning element 158 cooperating with the instrument axial positioning element 68 of the medical instrument 46 in the detection position.

The marker element axial positioning element 158 extends in the circumferential direction with respect to the marker element longitudinal axis 116 and points in a direction facing toward the shaft longitudinal axis 52 in the detection position.

The marker element axial positioning element 158 of the embodiment of the medical marker element 56 shown in Figures 1 to 15 is designed in the form of a projection 160. The projection 160 is designed in the form of an annular rib 162 protruding from an inner wall surface 164 of the sleeve wall 124.

In alternative embodiments not shown in the drawing Figures, the marker element axial positioning element 158 is designed in the form of a recess. The recess may, in particular, be designed in the form of an annular groove in the sleeve wall 124.

The annular rib 162 comprises four rib sections 166 which are separated by gaps 168 and 170. The gap 170 is arranged diametrically opposed to the opening 122 with respect to the marker element longitudinal axis 116.

The marker element 56 is of mirror symmetrical design. A symmetry plane 172 contains the marker element longitudinal axis 116. The two gaps 168 are arranged symmetrically with respect to the symmetry plane 172.

The annular rib 162 has a first side surface 174 and a second side surface 176. The first and second side surfaces 174, 176 face in opposite directions. The first side surface 174 faces in the distal direction, the second side surface 176 faces in the proximal direction.

The marker element axial positioning element 158 defines a second width 178 extending in the axial direction.

At least one clamping element 180 for clampingly engaging the instrument axial positioning element 68 is formed or arranged only on one of the first and second side surfaces 174, 176, namely only on the first side surface 174. In the embodiment shown in drawing Figures 1 to 15, two clamping elements 118 are arranged on the two rib sections 166 separated by gap 170.

The clamping elements 180 have a third width 182 extending in the axial direction. The marker element 56 is designed such that the first width 74 defined by the instrument axial positioning element 68 is smaller than the sum of the second and third widths 178 and 182. This sizing results in a clamping action such that the second side surface 176 is clampingly held against a first groove side surface 184 of the annular groove 72. The clamping element 180 is pressed against a second groove side surface 186 of the annular groove 72 and thereby deformed or compressed. As the clamping elements 180 are of weaker design than the annular rib 162, the clamping elements 180 are deformed and ensure a surface-to-surface contact between the annular rib 162, namely the second side surface 176 thereof, and the first groove side surface 184. Such a design of the marker element 56 ensures a defined axial position of the marker element 56 on the shaft portion 50 of the medical instrument 46.

The clamping elements 180 are designed in the form of clamping projections 188 having an end face 190 abutting against the second groove side surface 186. The clamping projection 188 extends away from the first side surface 174 on which it is arranged or formed.

The marker element axial positioning element 158 comprises at least two axial positioning element sections 192 which are spaced apart from each other in the circumferential direction. The axial positioning element sections 192 are formed by the rib sections 166.

The marker element interface 58 comprises a marker element circumferential positioning device 194 for positioning the marker element 46 in a defined circumferential position on the shaft portion 50. It comprises a marker element circumferential positioning element 196.

The marker element circumferential positioning device 194 cooperates with the instrument circumferential positioning device 104 of the medical instrument 46 in the detection position. Further, the marker element circumferential positioning element 196 cooperates with the instrument circumferential positioning element 106 in the detection position.

The marker element circumferential positioning element 196 is designed in the form of the recess 198, also called a notch. The recess 198 is open and faces in the proximal direction. Alternatively, a through opening can be provided as marker element circumferential positioning element 196 instead of the recess 198.

The marker element circumferential positioning device 194 comprises a first flap 200 projecting from the proximal end face 136 in the proximal direction. The recess 198 is formed on the first flap 200. The first flap 200 extends in the circumferential direction in an angular range of less than 180° and concentrically with respect to the marker element longitudinal axis 116.

A second flap 202 is arranged or formed on the sleeve wall 124, namely on the distal end face 138 and faces in the distal direction. The second flap 202 is recess-free.

The recess 198 is designed for receiving the corresponding projection 108 of the medical instrument 46. The projection 108 forms the instrument circumferential positioning element 106 as described above. Figure 4 depicts the detection position in which the projection 108 engages the recess 198. As only a single recess 198 is provided on the marker element 46, the marker element 56 can be connected to the medical instrument 46 in only a single and unique, and thus well-defined circumferential position.

As the second flap 202 does not have a recess, it cannot engage the pin 110 as is the case for the recess 198. This ensures that the marker element 56 can only be connected to the medical instrument 46 in a single orientation with respect to the shaft longitudinal axis 52.

The marker element 56 comprises at least three contact elements 204 and 206 for contacting the shaft 48 in the detection position. The contact elements 204, 206 are designed for cooperating with the abutment surfaces 100 and 102 of the instrument interface 54.

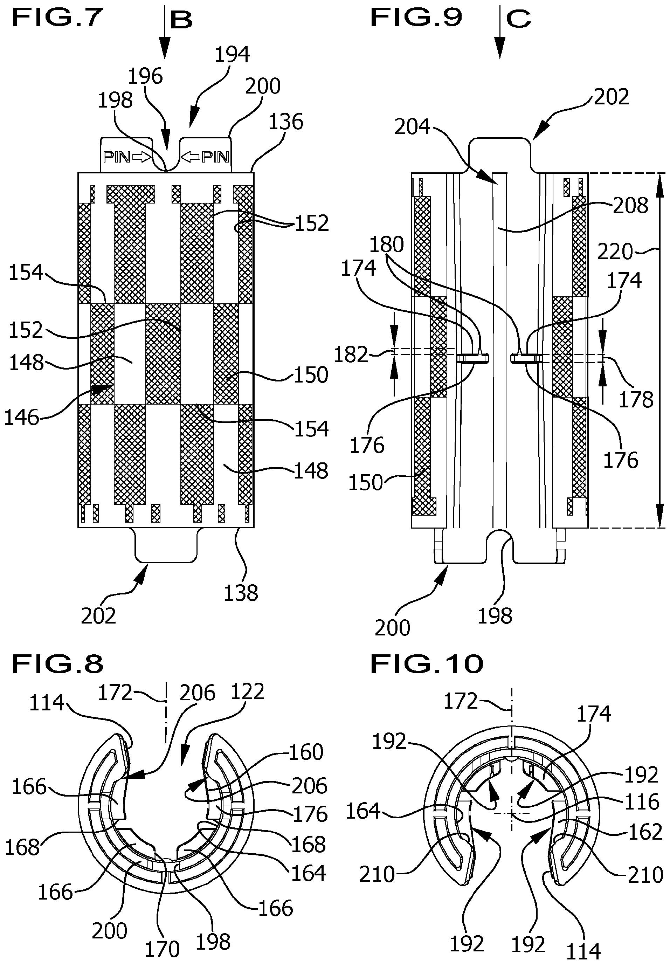

The contact elements 204 and 206 are in the form of projections 208 and 210 arranged or formed on the sleeve wall 124 and pointing in the direction toward the marker element longitudinal axis 116. The contact elements 204, 206 extend in parallel with the marker element longitudinal axis 116. As shown, in particular in Figures 3 and 9, the contact elements 204 and 206 form protruding ribs on the inner wall surface 164 over the entire length of the sleeve 112.

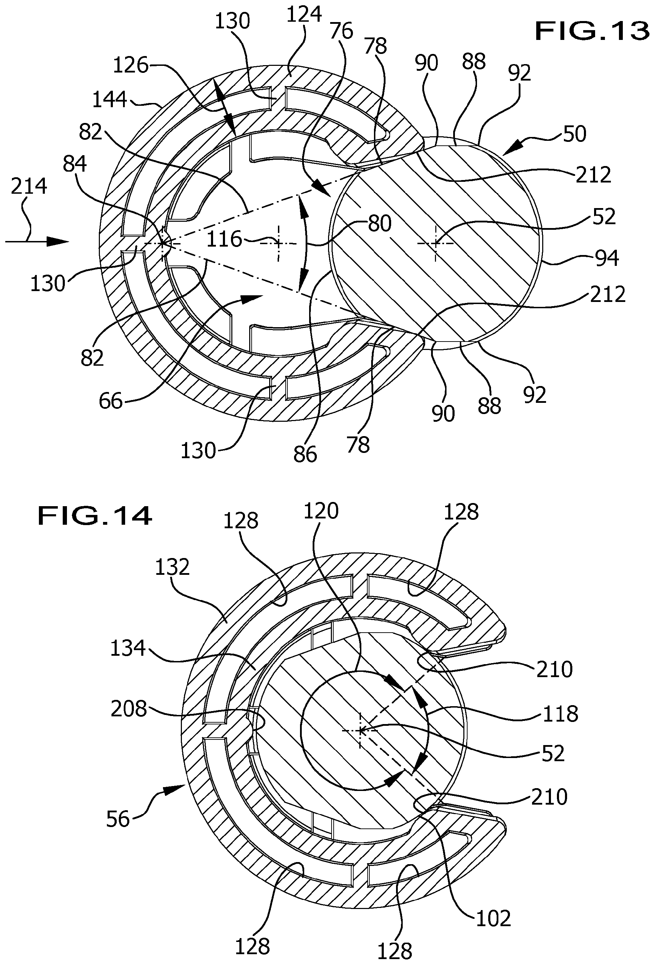

The embodiment of the marker element 56 depicted in Figures 1 to 15 is elastically deformable. It is expandable from a basic position shown, in particular, in Figures 7 to 10, to an expanded position, shown in particular in Figures 13 and 14. An outer diameter of the sleeve 112 is larger in the expanded position than in the basic position.

The contact element 204 is of mirror symmetrical design relative to the symmetry plane 172. It extends on the side of the sleeve 112 opposite the opening 122.

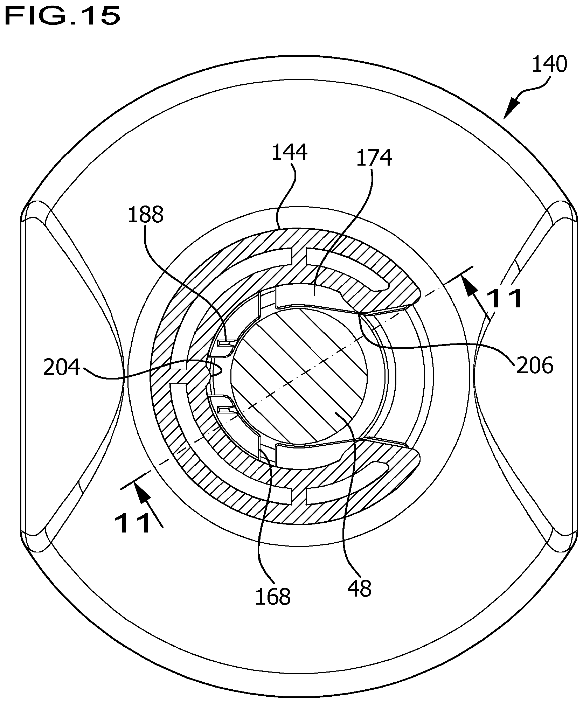

The two contact elements 206 are arranged on the inner wall surface 164 next to the opening 122. As shown, in particular, in Figures 14 and 15, the elastically deformable marker element 56 is clampingly held on the shaft portion 50 while the contact elements 204 and 206 are in line contact with the abutment surfaces 100 and 102 defined by the proximal and distal flanges 96 and 98.

The snap-fit connecting device 66 allows to connect the marker element 56 to the shaft 58 of the medical instrument 46 in a quick and simple manner as described as follows.

The sterile provided marker element 56 adopts the basic position before cooperating with the shaft 48. For connecting the marker element 56 to the shaft portion 50, the outer cylindrical surface portion 86 is introduced through the slot 114 toward the marker element longitudinal axis 116. The marker element 56 thereby contacts the first flat surfaces 78 with outer edges 212 of the outer sleeve wall 132. While pushing the marker element 56 in the direction of the arrow 214 shown in Figure 13, i.e. toward the shaft longitudinal axis 52, the outer edges 212 slide on the first flat surfaces 78 of the spreading device 76 and expand the sleeve 112 from the basic position into an expanded position. The maximum outer diameter of the sleeve 112 is defined by the marker element 56 contacting the two second flat surfaces 88. Once the marker element 56 is pushed forward over the edges 92, it snaps on the shaft portion 50 and the contact elements 206 come into contact with the outer cylindrical surface portion 94. At the same time, the contact element 204 comes into contact with the outer cylindrical surface portion 86.

The pin 110 is arranged on the proximal flange 96 such that it points away from the outer cylindrical surface portion 86, so that it can engage with the recess 98.

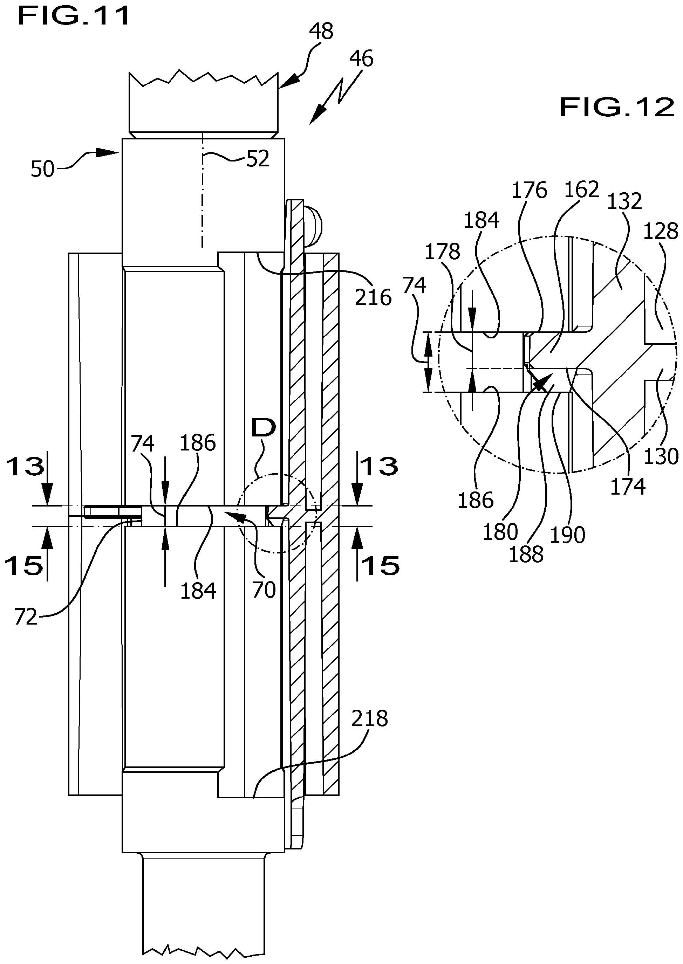

As the first flat surfaces 78 have a length in the axial direction such that the proximal and distal flanges 96 and 98 are partially removed over an angular range defined by the first and second flat surfaces 78 and 88 as depicted, in particular, in Figure 3, stop faces 216 and 218 are formed on the proximal and distal flanges 96, 98 pointing toward each other as shown, in particular, in Figures 3 and 11. This design facilitates spreading the sleeve 112 as the distance between the stop faces 216 and 218 is only slightly larger than a length 220 of the sleeve 112 extending in parallel to the marker element longitudinal axis 116. Further, this design of the first and second flat surfaces 78 and 88 facilitates and guides the introduction of the annular rib 162 into the annular groove 72. As already described, the clamping projections 108 force the second side surface 176 against the first groove side surface 184 for positioning the marker element 56 on the shaft portion 50 so as to be well-defined in the axial direction.

To remove the marker element 56, a user must simply exert a pushing force on the outer edges 212 in order to push the marker element 56 off the shaft portion 50.

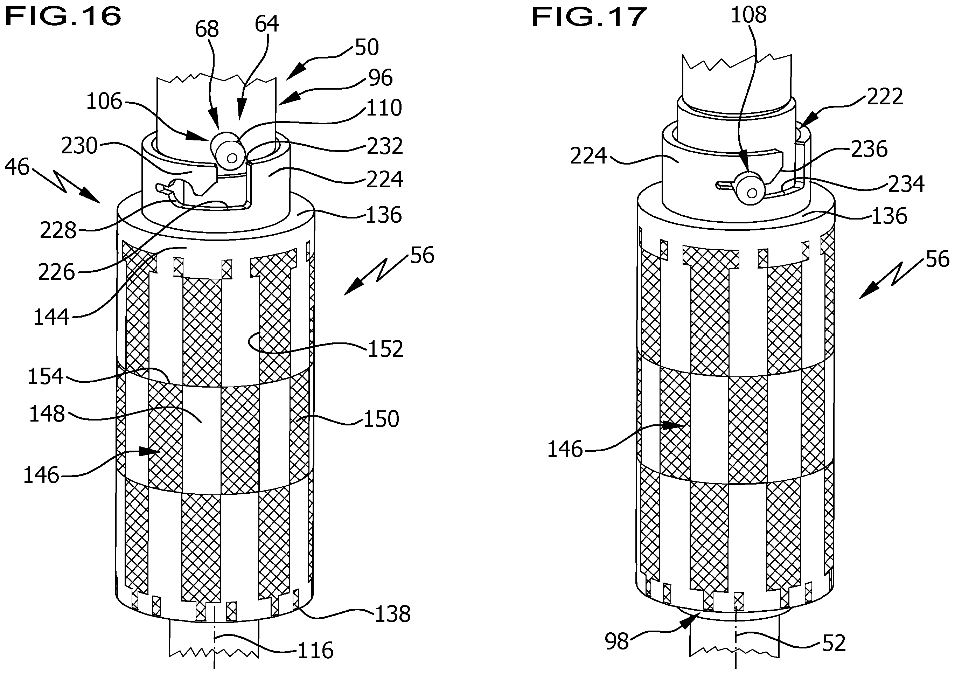

A further embodiment of a medical marker element is schematically depicted in Figures 16 and 17. It has a similar structure as the embodiment of the marker element 56 shown in Figures 1 to 15. Therefore, similar elements and functional equivalent components of the marker element 56 are provided with the same reference numerals.

The marker element 56 depicted in Figures 16 and 17 has a sleeve 112 which is completely closed. This means that the marker element 56 does not have a slot like the embodiment of Figures 1 to 15.

The marker element 56 is designed for the releasable connection to a shaft portion 50 of the medical instrument 46 described above.

The connecting device 64 for connecting the marker element 56 and the medical instrument 46 is in the form of a bayonet connecting device 222. In this embodiment, the bayonet connecting device 222 comprises the instrument circumferential positioning element 106 formed by the projection 108 in the form of the pin 110. The projection 108 also forms the instrument axial positioning element 68.

The sleeve 112 does not bear a flap or any other projection on the distal end face 138. However, a short closed bayonet sleeve 224 extends away from the proximal end face 136. The bayonet sleeve 224 has an outer diameter which is smaller than an outer diameter of the sleeve 112.

The bayonet sleeve 224 has a substantially L-shaped recess 226 with a first leg portion extending in parallel to the marker element longitudinal axis 116 and a second leg portion extending in the circumferential direction, i.e. perpendicularly to the marker element longitudinal axis 116. In the end region of the leg extending in the circumferential direction, a receptacle 228 is formed for receiving the projection 108 in a form- and force-locking manner in the detection position as shown in Figure 17. The receptacle 228 is partially delimited by a flexible leg 230 which allows a snap-fit connection with the projection 108 in the detection position.

The bayonet sleeve 224 has an inner diameter which corresponds substantially with an outer diameter of the proximal flange 96.

The sleeve of the marker element depicted in Figures 16 and 17 also has contact elements 204 and 206 as described in connection with the embodiment of Figures 1 to 15. This allows for a three point bearing or three line bearing on the proximal and distal flanges 96 and 98.

For connecting the marker element 56 to the shaft portion 50, the instrument tip 60 is introduced through the bayonet sleeve 224 and the marker element 56 is pushed toward the pin 110. Then, the pin 110 is introduced into an opening 232 of the L-shaped recess 226 pointing in the proximal direction and is moved in the distal direction relative to the marker element 56 until the pin 110 abuts an edge 234 of the leg portion extending in the circumferential direction.

For bringing the marker element 56 both in an axially and circumferentially defined detection position, the marker element 56 has to be rotated relative to the shaft portion 50 about the shaft longitudinal axis 52 until the pin 110 abuts against an inclined sliding surface 236 on the leg 230 which causes the leg 230 to deflect in the proximal direction so that the pin 110 can enter the receptacle 228. Once the pin 110 is received in the receptacle 228, the leg 230 snaps back into its original position shown in Figure 16 and holds the pin 110 clampingly in the receptacle 228 as shown in Figure 17.

For determining the position and/or orientation of the marker element 56 in space, the outer surface 144 of the sleeve 112 is provided with a pattern 146 of a design as described with the embodiment depicted in Figures 1 to 15.

For removing the marker element 56 from the medical instrument 46, a user has to rotate the marker element 56 in the opposite direction as for connecting the same to the medical instrument 46 so that the pin 110 can be moved out of the L-shaped recess 226 through the opening 232.

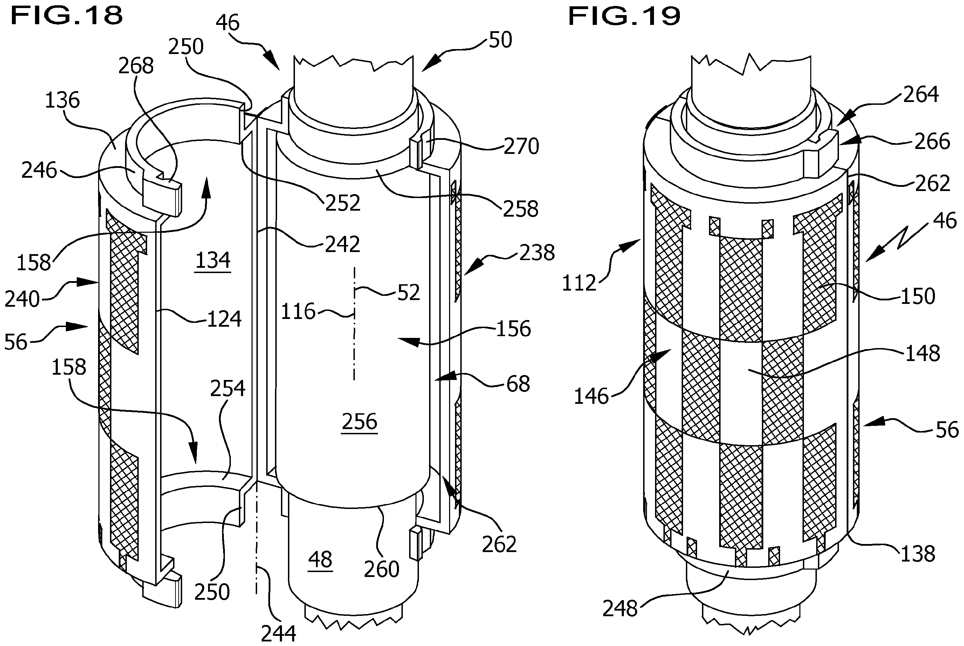

A further embodiment of a medical marker element denoted with reference numeral 56 is shown in Figures 18 and 19. Again, the same reference numerals are used for denoting identical or functionally equivalent elements and components of the marker element 56 and the medical instrument 46 shown and described in connection with Figures 1 to 15.

The marker element 56 comprises a sleeve 112 which comprises two sleeve portions 238 and 240 in the form of half shells. The sleeve portions 238 and 240 are connected to one another by means of an integral hinge 242. It allows for rotating the two sleeve portions 238 and 240 relative to one another about a hinge axis 244 extending in parallel to the marker element longitudinal axis 216.

Both the proximal end face 136 and the distal end face 138 comprise short connecting sleeve 246 and 248, extending in the proximal direction on the one hand and in the distal direction on the other hand. An outer diameter of the connecting sleeves 246 and 248 is smaller than an outer diameter of the sleeve 112. As the sleeve wall 124 has the same thickness as a wall 250 of the connecting sleeves 246 and 248, stop surfaces 252 and 254 are formed. The stop surface 252 faces in the distal direction and the stop surface 254 faces in the proximal direction. Stop surfaces 252 and 254 form part of an inner sleeve wall 134.

The stop surfaces 252 and 254 form marker element axial positioning elements 158 of a marker element axial positioning device 156. A corresponding instrument axial positioning element 68 is formed by an annular flange 256 on the shaft 48. The flange 56 has an outer diameter which is larger than an outer diameter of the shaft 48. This results in stop surfaces 258 and 260 facing in the proximal direction and in the distal direction. They cooperate with the stop surfaces 252 and 254 in the detection position. A distance between the stop surfaces 258 and 260 corresponds to a distance between the stop surfaces 252 and 254. This results in a defined axial positioning of the marker element 56 on the shaft portion 50.

The two sleeve portions 238 and 240 are arranged so as to be movable relative to each other as already described. The two sleeve portions 238 and 240 can be brought from a first position, in which they are separated by at least one slot 262 as shown in Figure 18, to a second position, in which the at least one slot 262 is closed so that the two sleeve portions 238 and 240 form a closed sleeve 112. This design of the marker element 56 allows for it to be connected to the shaft portion 50 by introducing the flange 256 in one of the two sleeve portions 238, 240 as shown in Figure 18. For securing the marker element 56 on the shaft portion 58, the marker element 56 comprises a locking device 264 for locking the two sleeve portions 238 and 240 in the second position.

The locking device 264 is designed in the form of a latching device 266. The latching device 266 comprises cooperating latching elements 268 and 270 formed on the connecting sleeves 246 and 248 on each of the two sleeve portions 238 and 240. They are designed in the form of latching noses and latching recesses which are in engagement in the second position and out of engagement in the first position. The latching device 266 enables the releasable connection of the marker element 56 and the medical instrument 46.

The sleeve 112 also bears a pattern 146 on the outer surface 144 as described above. As the embodiment of the marker element 56 depicted in Figures 18 and 19 does not have a marker element circumferential positioning device, the sleeve 112 can rotate about the shaft longitudinal axis 52 about the shaft portion 50. Therefore, this design allows only determining a position of the medical instrument 46 in space but not its orientation as only the axial position of the marker element 56 on the medical instrument 46 is predetermined but not a circumferential position thereof.

The embodiments of marker elements 56 described above are all made from one piece. Preferably, they are made monolithically.

The marker elements 56 are made of a sterilizable plastic material. In particular, the plastic material can be sterilizable by beta radiation, gamma radiation or overheated steam.

The medical marker elements described above are in the form of single use medical marker elements 56. This means that they can be simply disposed of after being used in a surgical procedure. Cleaning and sterilizing the marker elements 56 after use is not required.

The embodiment of the marker element 56 depicted in Figures 1 to 15 allows a precise snap-fit assembly without unintended rotation of the marker element 56 as would occur without the two first flat surfaces 78. Advantages of this design are an intuitive assembly, a strong and safe connection with good protection against unintended rotational and axial movement, and, in particular, no design restrictions regarding the instrument's working ends, as the marker element does not have to pass over the working end as is the case for the embodiment shown in Figures 16 and 17.

Although the pattern 146 only covers an angular range of about 270°, this is not limiting and/or disturbing to a work flow when tracking the medical instrument 46 bearing the embodiment of the marker element 56 depicted in Figures 1 to 15.

The bayonet connecting device 222 also allows an intuitive assembly. However, it does not have a protection against unintended rotational movement. In case of the recess 226 being of U-shaped design, no protection against an unintended movement would be provided. A further disadvantage of the embodiment shown in Figures 16 and 17 is that such a marker element 56 can only be used with instrument working ends that allow passing the sleeve 112 over the respective working ends. Therefore, design restrictions regarding the instrument working ends apply to the bayonet connecting device 222.

The marker element 56 of the embodiment shown in Figures 18 and 19 has the advantage that a pattern visible over 360° does not provide any line-of-sight issues which means that no special alignment to the cameras 32 is required. This also applies to the embodiment shown in Figures 16 and 17.

A defined circumferential positioning of the marker element 56 on the shaft 48 can be made possible if a projection 108 is provided on the shaft portion 50 which cooperates with a corresponding recess 198 on one of the two connecting sleeves 246 and 248.

All described embodiments of marker elements 56 are lightweight, can be manufactured at low costs, but still allow sufficient accuracy in determining their position and/or orientation in space so that a position and/or orientation of a medical instrument 46 provided with a marker element 56 can be determined in space with high accuracy.

The assembly of the medical instrument 46 and the marker element 56 is quick and easy to handle for a user.

### Reference numeral list

- 10: medical system
- 12: surgeon
- 14: referencing unit
- 16: carrier element
- 18: marker element
- 20: sphere
- 22: medical instrument
- 24: palpation instrument
- 26: tip
- 28: optical detection unit
- 30: radiation source
- 32: camera
- 34: computer
- 36: display
- 38: patient
- 40: hip
- 42: referencing unit
- 44: bone fastener
- 46: medical instrument
- 48: shaft
- 50: shaft portion
- 52: shaft longitudinal axis
- 54: instrument interface
- 56: medical marker element
- 58: marker element interface
- 60: instrument tip
- 62: distal end
- 64: connecting device
- 66: snap-fit connecting device
- 68: instrument axial positioning element
- 70: recess
- 72: annular groove
- 74: first width
- 76: spreading device
- 78: first flat surface
- 80: spreading angle
- 82: first flat plane
- 84: line
- 86: outer cylindrical surface
- 88: second flat surface
- 90: edge
- 92: edge
- 94: outer cylindrical surface portion
- 96: proximal flange
- 98: distal flange
- 100: abutment surface
- 102: abutment surface
- 104: instrument circumferential positioning device
- 106: instrument circumferential positioning element
- 108: projection
- 110: pin
- 112: sleeve
- 114: slot
- 116: marker element longitudinal axis
- 118: slot angle
- 120: sleeve angle
- 122: lateral opening
- 124: sleeve wall
- 126: wall thickness
- 128: wall opening
- 130: web
- 132: outer sleeve wall
- 134: inner sleeve wall
- 136: proximal end face
- 138: distal end face
- 140: handle
- 142: proximal end
- 144: outer surface
- 146: optical pattern
- 148: light region
- 150: dark region
- 152: border line
- 154: border line
- 156: marker element axial positioning device
- 158: marker element axial positioning element
- 160: projection
- 162: annular rib
- 164: inner wall surface
- 166: rib section
- 168: gap
- 170: gap
- 172: symmetry plane
- 174: first side surface
- 176: second side surface
- 178: second width
- 180: clamping element
- 182: third width
- 184: first groove side surface
- 186: second groove side surface
- 188: clamping projection
- 190: end face
- 192: axial positioning element section
- 194: marker element circumferential positioning device
- 196: marker element circumferential positioning element
- 198: recess
- 200: first flap
- 202: second flap
- 204: contact element
- 206: contact element
- 208: projection
- 210: projection
- 212: outer edge
- 214: arrow
- 216: stop face
- 218: stop face
- 220: length
- 222: bayonet connecting device
- 224: bayonet sleeve
- 226: recess
- 228: receptacle
- 230: leg
- 232: opening
- 234: edge
- 236: sliding surface
- 238: sleeve portion
- 240: sleeve portion
- 242: integral hinge
- 244: hinge axis
- 246: connecting sleeve
- 248: connecting sleeve
- 250: wall
- 252: stop surface
- 254: stop surface
- 256: flange
- 258: stop surface
- 260: stop surface
- 262: slot
- 264: locking device
- 266: latching device
- 268: latching element
- 270: latching element

## Claims

1. Medical marker element (56) which is formed for the releasable connection to a medical instrument (46), the medical marker element (56) being optically detectable for determining a position and/or orientation of the medical marker element (56) in space, in particular in an operating room, wherein the medical marker element (56) comprises a marker element interface (58) which is formed for the connection to a corresponding instrument interface (54) of the medical instrument (46) in a detection position, wherein the marker element interface (58) and the instrument interface (54) are in engagement in the detection position in a defined manner and are out of engagement in a cleaning position, **characterized in that** the medical marker element (56) comprises a sleeve (112) surrounding a shaft portion (50) of a shaft (48) of the medical instrument (22).

2. Medical marker element in accordance with claim 1, **characterized in that** the marker element interface (58) comprises a marker element axial positioning device (156) for positioning the medical marker element (56) in a defined axial position on a shaft portion (50) of a shaft (48) of the medical instrument (46) defining a shaft longitudinal axis (52) thereof, wherein, in particular, the marker element axial positioning device (156) comprises at least one marker element axial positioning element (158) cooperating with an instrument axial positioning element (68) of the medical instrument (46) in the detection position.

3. Medical marker element in accordance with any one of the preceding claims, **characterized in that** the marker element interface (58) comprises a marker element circumferential positioning device (194) for positioning the medical marker element (46) in a defined circumferential position on a shaft portion (50) of a shaft (48) of the medical instrument (46) defining a shaft longitudinal axis (52) thereof,
wherein, in particular, the marker element circumferential positioning device (194) comprises at least one marker element circumferential positioning element (196) cooperating with an instrument circumferential positioning device (104) of the medical instrument (46), in particular at least one instrument circumferential positioning element (106), in the detection position.

4. Medical marker element in accordance with any one of the preceding claims, **characterized in that** the marker element (56) comprises at least three contact elements (204, 206) for contacting the shaft (48) in the detection position, in particular on corresponding abutment surfaces (100, 102) of the instrument interface (54),
wherein, at least one of
a) the at least three contact elements (204, 206) are in the form of projections (208, 210) arranged or formed on the sleeve wall (124) and pointing in the direction toward a marker element longitudinal axis (116) of the marker element (56)
and
b) the at least three contact elements (204, 206) extend in parallel with the marker element longitudinal axis (116).

5. Medical marker element in accordance with any one of the preceding claims, **characterized in that** the marker element (56), in particular the sleeve (112), is expandable from a basic position to an expanded position, an outer diameter of the marker element (56) being larger in the expanded position than in the basic position.

6. Medical marker element in accordance with any one of the preceding claims, **characterized in that** the marker element (56), in particular the sleeve (112), comprises a slot (114) extending in a direction parallel or substantially parallel to a marker element longitudinal axis (116) of the marker element (56).

7. Medical marker element in accordance with any one of the preceding claims, **characterized in that** the sleeve (112) is completely closed, in particular in the basic position.

8. Medical marker element in accordance with any one of the preceding claims, **characterized in that** the sleeve (112) comprises at least two sleeve portions (238, 240) which are arranged so as to be movable relative to each other, **in that** the at least two sleeve portions (238, 240) can be brought from a first position, in which they are separated by at least one slot (262), to a second position, in which the at least one slot (262) is closed so that the at least two sleeve portions (238, 240) form a closed sleeve (112),
wherein, in particular, the marker element comprises a locking device (264) for locking the at least two sleeve portions (238, 240) in the second position.

9. Medical marker element in accordance with any one of the preceding claims, **characterized in that** the marker element interface (58) comprises a bayonet connecting element (222) corresponding to an instrument connecting element of the instrument (46),
wherein, in particular, the bayonet connecting element (222) comprises the marker element axial positioning device (156) and the marker element circumferential positioning device (194).

10. Medical marker element in accordance with any one of the preceding claims, **characterized in that** the outer surface (144) of the sleeve (112) is provided with an optically visible pattern (146) which is detectable by an optical detection device (28), in particular a camera (32), wherein, in particular, at least one of
a) the optical pattern (144) comprises light and dark, in particular black and white, regions (148, 150), in particular in the form of polygons, further in particular in the form of rectangles,
and
b) the medical marker element (56) defines a marker element longitudinal axis (116) and wherein the optical pattern (146) defines border lines (152, 154) along light and dark regions (148, 150) next to each other, and wherein the border lines (152) extend in parallel with the marker element longitudinal axis (116) or in a circumferential direction with respect to the marker element longitudinal axis (116).

11. Medical marker element in accordance with any one of the preceding claims, **characterized in that** the marker element (56) at least one of
a) is made from one piece, in particular monolithically and
b) is made of a sterilizable plastic material and
c) is in the form of a single use medical marker element (56).

12. Medical instrument (46) comprising a shaft (48), wherein the shaft (48) has a shaft portion (50), which defines a shaft longitudinal axis (52) of the instrument (46), and wherein the medical instrument (46) has an instrument interface (54) for releasably connecting to a medical marker element interface (58) of a medical marker element (56), which is optically detectable for detecting a position and/or orientation of the medical marker element (56) and the medical instrument (46) connected thereto in space, in particular in an operating room, wherein the instrument interface (54) and the marker element interface (58) are in engagement in a detection position in a defined manner and are out of engagement in a cleaning position, **characterized in that** the instrument interface (54) is formed on the shaft portion (50) and designed for connecting to the marker element interface (58) of a medical marker element (56) in accordance with any one of the preceding claims,
wherein the medical instrument interface (54) and the marker element interface (58) form a connecting device (64) for the at least one of force locking and form locking connection of the medical instrument (46) and the medical marker (56) element in the detection position.

13. Medical instrument in accordance with claim 12 , **characterized in that** the instrument interface (54) at least one of
a) comprises an instrument axial positioning element (68) which cooperates with the marker element axial positioning element (156) in the detection position for positioning the medical marker element (56) on the medical instrument (46) in a defined axial position on the shaft portion (50),
wherein, in particular, the instrument axial positioning element (68) extends in the circumferential direction with respect to the shaft longitudinal axis (52) and points in a direction facing away from the shaft longitudinal axis (52),
and
b) comprises a spreading device (76) for spreading the sleeve of the medical marker element when connecting to the shaft, wherein, in particular, the spreading device (76) comprises two first flat surfaces (78) which are formed or arranged on the shaft (48) so as to define a spreading angle (80) between them,
and
c) comprises a proximal flange (96) and a distal flange (98) arranged or formed on the shaft portion (50), and the proximal and distal flanges (96, 98) define abutment surfaces pointing in radial direction for the contact elements (204, 206) of the marker element (56),
wherein, in particular, the abutment surfaces (100, 102) are in the form of cylindrical surfaces or portions of cylindrical surfaces, and **in that** the cylindrical surfaces are concentric with respect to the shaft longitudinal axis (52),
and
d) comprises an instrument circumferential positioning device (104) cooperating with the marker element circumferential positioning device (194) in the detection position,
wherein, in particular, the instrument circumferential positioning device (104) comprises at least one instrument circumferential positioning element (166) which is designed in the form of a projection (108) or recess pointing in a radial direction with respect to the shaft longitudinal axis (52), in particular pointing away from the shaft longitudinal axis (52).

14. Medical system (10) comprising at least one medical instrument (46) and at least one medical marker element (56), the at least one marker element (56) being releasably connectable to the at least one medical instrument (46) in a detection position for optically tracking the medical instrument (46), in particular its position and/or orientation in space, in particular in an operating room, **characterized in that** the medical instrument (46) is in the form of a medical instrument (46) in accordance with claim 12 or 13, and **in that** the at least one medical marker element (56) is in the form of a medical marker element (56) in accordance with any one of claims 1 to 11.

15. Medical system (10) in accordance with claim 13, **characterized in that** the medical system (10) comprises an optical detection device (28) for optically detecting the marker element, and the optical detection device (28) is designed for determining at least one of a position and orientation of the medical marker element ((56) in space, in particular in an operating room.
